# EUROPEAN PATENT SPECIFICATION

(11) **EP 3 846 827 B1**
(45) Date of publication and mention of the grant of the patent: **28.10.2026**
(21) Application number: 19768918.5
(22) Date of filing: 04.09.2019
(51) Int. Cl.: A61K 33/42, A61K 47/00, A61P 35/00, A61P 35/04, A61K 9/00, A61K 9/14

(54) **2D EXFOLIATED BLACK PHOSPHORUS FOR USE IN THE TREATMENT OF BONE CANCER WITHOUT PHOTOTHERAPY**
2D-EXFOLIERTER SCHWARZER PHOSPHOR ZUR VERWENDUNG BEI DER BEHANDLUNG VON KNOCHENKREBS OHNE PHOTOTHERAPIE
PHOSPHORE NOIR EXFOLIÉ 2D POUR LE TRAITEMENT DU CANCER DES OS SANS PHOTOTHÉRAPIE

(30) Priority: 06.09.2018 IT 201800008405
(43) Date of publication of application: 14.07.2021
(73) Proprietor: Materias S.r.l., 80122 Napoli (IT); Raucci, Maria Grazia, 81025 Marcianise (CE) (IT); Ambrosio, Luigi, 80044 Ottaviano (NA) (IT); Fasolino, Ines, 81100 Caserta (IT); Caporali, Maria, 50019 Sesto Fiorentino (FI) (IT); Serrano Ruiz, Manuel, 50019 Sesto Fiorentino (FI) (IT); Peruzzini, Maurizio, 50018 Scandicci (FI) (IT)
(72) Inventor: RAUCCI, Maria Grazia, 81025 Marcianise (CE) (IT); AMBROSIO, Luigi, 80044 Ottaviano (NA) (IT); FASOLINO, Ines, 81100 Caserta (IT); CAPORALI, Maria, 50019 Sesto Fiorentino (FI) (IT); SERRANO RUIZ, Manuel, 50019 Sesto Fiorentino (FI) (IT); PERUZZINI, Maurizio, 50018 Scandicci (FI) (IT)
(74) Representative: PGA S.p.A.
(86) International application number: PCT/IB2019/057444
(87) International publication number: WO 2020/049475

(56) References cited:
- CN-A- 105 535 971
- CN-A- 106 668 951
- CN-A- 107 936 266
- CN-A- 108 030 919
- XING CHENYANG ET AL: "Conceptually Novel Black Phosphorus/Cellulose Hydrogels as Promising Photothermal Agents for Effective Cancer Therapy.", ADVANCED HEALTHCARE MATERIALS APR 2018, vol. 7, no. 7, April 2018 (2018-04-01), pages e1701510, XP002791459, ISSN: 2192-2659
- YANG BOWEN ET AL: "2D-Black-Phosphorus-Reinforced 3D-Printed Scaffolds:A Stepwise Countermeasure for Osteosarcoma.", ADVANCED MATERIALS (DEERFIELD BEACH, FLA.) MAR 2018, vol. 30, no. 10, March 2018 (2018-03-01), XP002791460, ISSN: 1521-4095

## Description

### FIELD OF THE INVENTION

The present invention relates to exfoliated black phosphorus for use in the treatment of a bone cancer, in particular but not limited to osteosarcoma, without any irradiation by photothermal or photodynamic therapy, and to a pharmaceutical composition for parenteral administration comprising exfoliated black phosphorus.

### BACKGROUND OF THE INVENTION

Most patients with cancer die not because of the tumor in the primary site, but generally because it has spread to other sites. Patients with advanced breast and prostate cancers may often develop bone metastases. Bone metastases are infrequently silent. They are usually associated with severe bone pain for which the therapeutic approach is based only on analgesic drugs. The mechanisms responsible for bone pains are poorly understood, however it seems to be a consequence of osteolysis (bone brittleness) leading to an increase of fractures. Pathological fractures frequently occur as a consequence of bone metastases [G.R. Mundy, Nat Rev. Cancer. 2002, 2(8), 584].

Nowadays, several treatments are available: (a) irradiation, (b) surgery, (c) use of bisphosphonates, and (d) rarely chemotherapy and hormonal therapy as an adjuvant in well-defined tumor types. A fifth possibility is a combination of all the above ones. However, these procedures are based on tumor removal by surgery or by physical and chemical (irradiation, chemotherapy) treatments [M. Aebi, Eur Spine J. 2003, 12, S202-13]. The latter lacks of selectivity between healthy and cancer cells during the inhibition of cell division. Furthermore, there is a great and growing needs to repair and/or regenerate bone tissue to prevent or treat secondary fractures such as vertebrae burst fractures.

Currently, osteosarcoma is the most common bone cancer which mainly affects young people [A. Longhi et al., Cancer Treat Rev. 2006, 32, 423-436]. Surgical resection of tumor followed by chemotherapy for micro-metastasis inhibition constitutes the current standard procedure to cure 65% of osteosarcoma cases. However, osteosarcoma often is refractory to standardized chemotherapy regime. Chemotherapy, on the other hand, uses chemotherapeutic drugs with the effect of blocking cell proliferation, without any distinction between healthy and cancer cells [S. Ferrari, Chemotherapy in Osteosarcoma. Atlas of Musculoskeletal Tumors and Tumor like Lesions pp 189-193 in Ed. Springer 2014]. In fact, systemic chemotherapy may also cause toxicity to healthy cells that are not affected by cancer.

Thus, many efforts have been undertaken to identify novel strategies for the osteosarcoma treatment. Besides the new pharmacological findings of novel drugs such as Sorafenib (Nexavar^{®}), current approaches are aimed at investigating the use of nanomedicines for osteosarcoma therapy. Several studies are based on the opportunity to release chemotherapeutic drugs in local site creating the potential to improve both the safety and efficacy of cancer chemotherapy.

In particular, the introduction of a suitable material shows the advantage to improve the benefit of surgery by minimizing the systemic toxicity that is usually associated with standard pharmacological treatments. The possibility to promote the anticancer drug release by using external stimuli such as magnetic, electrical stimuli or by using a hyperthermia treatment is well known in literature [J. Gupta et al., J. Magn. Magn. Mater. 2018, 448, 332-338 e L. Gengci et al., J. Sol-Gel Sci. Technol. 2015, 75, 90-97]. However, local release of an anticancer drug does not overcome the drawback related to the distinction of cancer from healthy cells. In recent years, several studies have been focused on the use of photodynamic therapy (PDT) as a minimally invasive therapeutic procedure that can apply a selective cytotoxic activity toward cancer cells [R. Allison Ron et al., Clin. Endosc. 2013, 24-29 e J.M. D 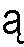 browski et al., *Photochem. Photobiol. Sci.* **2015,** 14, 1765-1780].

On this respect, a recently discovered 2D material, named phosphorene, being the P-counterpart of graphene and obtained by exfoliation of crystals of the black allotrope of the element, has attracted the attention of scientists working in the biomedical field intrigued by its very low (or absent) toxicity towards various cells [N. M. Latiff et al., *Chem. Eur.*

*J.* **2015,** 21, 13991-13995]. Being phosphorene the monolayer, the 2D nanomaterial usually employed in the chemical, physical and biomedical applications, is the exfoliated black phosphorus, also named 2D black phosphorus or "2D bP". To date exfoliated black phosphorus, as a novel two-dimensional nano-material, has attracted great attention owing to its anticancer properties in cancer phototherapy, namely photothermal therapy (PTT) or photodynamic therapy (PDT). Recent studies have shown the effectiveness of 2D bP as photodynamic therapy agent for cancer treatment [J. Shao et al., Nat. Commun. 2016, 7, 12967 and Yang Bowen et al., "2D-Black-Phosphorus-Reinforced 3D-Printed Scaffolds:A Stepwise Countermeasure for Osteosarcoma.", Advanced Materials, 2018, 30(10), ISSN: 1521-4095). This activity has been ascribed to the capability of 2D bP to generate singlet oxygen and to act as photosensitizer that, in presence of reactive oxygen species (ROS) and infrared light irradiation, constitutes an essential component of PDT therapy. Notably it is reported that after a near-infrared light irradiation of ultrathin bP nanosheets, singlet oxygen is generated that serves as effective photodynamic therapy (PDT) in cancer treatment [W. Tao et al., Adv Mater. 2017, 29, 1603276].

### SUMMARY OF THE INVENTION

The Applicant has surprisingly found that 2D exfoliated black phosphorus has effect on bone cancer even in the absence of irradiation by photothermal or photodynamic therapy.

The Applicant has found that the presence of 2D exfoliated black phosphorus without irradiation inhibits the metabolic activity of osteosarcoma cells (SAOS-2), while inducing both the proliferation and the osteogenic differentiation of human pre-osteoblast cells (HOb) and mesenchymal stem (hMSC).

In particular, the results obtained by the Applicant suggest that 2D exfoliated black phosphorus not only contributes to inhibit cancer progression but also induces new bone tissue formation due to its oxidation to phosphate anions that, combined with calcium cations, represent hydroxyapatite (the major component of bone) precursors.

Thus, the Applicant has found that 2D exfoliated black phosphorus exerts anti-proliferative effects on cancer cells and simultaneously promotes induction of newly forming bone tissue development by stimulating pre-osteoblast differentiation.

Further, the Applicant has found that the presence of 2D exfoliated black phosphorus was able to inhibit the ALP expression in SAOS-2 cells, thus indicating the ability of 2D exfoliated black phosphorus as substrate to block the metabolic activity of osteosarcoma derived cancer cells.

The Applicant also observed that the measurement of ROS production after 24 hours due to the stimulation caused by the Fenton reagent, indicated that on healthy HOb the presence of 2D exfoliated black phosphorus without irradiation reduced the ROS production, suggesting potential antioxidant properties. On the contrary, the presence of 2D exfoliated black phosphorus doubled the ROS production of osteosarcoma derived cells (SAOS-2), compared to the reference experiment.

In other words, the Applicant has surprisingly found that 2D exfoliated black phosphorus (i) induces anti-proliferative and apoptotic effects, without any phototherapy treatment, by increasing the production of ROS on osteosarcoma cell line (SAOS-2), and at the same time (ii) shows an opposite behavior in the case of healthy human osteoblasts (HOb) and mesenchymal stem cells (hMSC) by inducing cell proliferation, osteogenic marker expression and a protective effect against oxidative stress species, thus making 2D exfoliated black phosphorus a powerful material in cancer therapy.

Finally, the Applicant has found that in an *in vitro* co-culture model (SAOS-2 and HOb cell lines) 2D exfoliated black phosphorus may increase anti-inflammatory cytokines generation (i.e. interleukin-10) and inhibit pro-inflammatory mediators synthesis (i.e. interleukin-6), thus suggesting the opportunity to prevent cancer-related inflammation. Then, the Applicant's results suggest the use of 2D exfoliated black phosphorus in the treatment of bone cancer as anti-cancer agent with osteoinductive properties, different from the use as carrier for anti-cancer agents. Accordingly, a first object of the present invention relates to 2D exfoliated black phosphorus for use in the treatment of bone cancer without phototherapy.

Additionally, the Applicant has found that 2D exfoliated black phosphorus can be formulated as an injectable composition comprising inorganic salts, such as calcium phosphate, and/or polymeric materials, such as gelatin, hyaluronic acid, cellulose or chitosan.

Then, a second aspect of the present invention is a pharmaceutical composition for parenteral administration comprising comprising an injectable bio-composite of 2D exfoliated black phosphorus (2D bP) with hydroxyapatite and a polymeric material selected from the group consisting of hyaluronic acid, collagen, fibronectin, laminin and vitronectin.

### BRIEF DESCRIPTION OF THE FIGURES

Figure 1 shows a SEM image of bulk black phosphorus.
Figure 2 shows a bright field TEM image of exfoliated black phosphorus with scale bar of 1000 nm.
Figure 3 shows the effect of red and black phosphorus in DMSO at different concentration on L929 (murine fibroblasts) viability after 1 and 2 days of exposure as described in Example 2.1.
Figure 4 shows the effect of red and black phosphorus in DMSO at different concentration on MSC viability after 1 and 2 days of exposure as described in Example 2.1.
Figure 5 shows the effect of 2D bP on MSC viability after 3, 7 and 14 days of exposure (A) and the alkaline phosphatase activity (B) results after 3, 7, 14 and 21 days of cell cultures as described in Example 2.1.
Figure 6 shows Alamar blue (A) and alkaline phosphatase activity (B) results after 3, 7, 14 and 21 days of healthy HOb cell cultures as described in Examples 2.2 and 2.3.
Figure 7 shows Alamar blue (A) and alkaline phosphatase activity (B) results after 3, 7, 14 and 21 days of osteosarcoma SAOS-2 cell cultures as described in Examples 2.2 and 2.3.
Figure 8 shows SEM images after seven days of cell culture by using HOb (A-C) and SAOS-2 (D-F) cells on 2D bP substrate as described in Example 2.4.
Figure 9 shows confocal images of HOb (A, B) and SAOS-2 cells on 2D bP substrates (C, D) obtained using immunofluorescence analysis after 7 days of culture as described in Example 2.5.
Figure 10 shows Alamar blue results after 2 cycles of infrared treatment on HOb (A) and SAOS-2 (B) as described in Example 2.6.
Figure 11 shows Reactive Oxygen Species (ROS) production from HOb (A-B) and SAOS-2 (C-D) cells with and without infrared stimulation as described in Example 2.6.
Figure 12 shows the effect of irradiated and not irradiated 2D bP on interleukin-6 (IL-6) (A,B) levels in a co-culture in *vitro* model (SAOS-2 plus HOb) treated with lipopolysaccharide (LPS) as described in Example 2.7.
Figure 13 shows the effect of irradiated and not irradiated 2D bP on interleukin-10 (IL-10) (A,B) levels in a co-culture *in vitro* model (SAOS-2 plus HOb) treated with lipopolysaccharide (LPS) as described in Example 2.7.
Figure 14 shows the effect of not irradiated 2D bP on nitrites levels in a co-culture *in vitro* model (SAOS-2 plus HOb) treated with lipopolysaccharide (LPS) as described in Example 2.8.

### DETAILED DESCRIPTION OF THE INVENTION

According to the first object, the present invention relates to 2D exfoliated black phosphorus for use in the treatment of bone cancer without phototherapy.

2D exfoliated black phosphorus is able to inhibit the proliferation and viability of bone cancer cells and at the same time is able to promote the proliferation and viability of healthy bone cells.

Bone cancer cells include primary bone cancer cells such as osteosarcoma cells, cells from Ewing's family of tumors, chondrosarcoma cells, malignant giant cell tumor cells, malignant fibrous histiocytoma cells, and adamantinoma cells, as well as secondary bone cancer cells that have metastasized from other tissues, including breast, lung, prostate, and kidney.

2D exfoliated black phosphorus can be obtained by liquid exfoliation of bulk crystalline black phosphorus under the action of ultrasounds following the procedure disclosed by M. Serrano-Ruiz et al., Adv. Mater. Interfaces 2016, 3, 1500441. Bulk crystalline black phosphorus can be prepared from red phosphorus according to the procedure disclosed by T. Nilges et al., J. Solid State Chem. 2008, 181, 1707-1711.

Generally, bulk crystals of black phosphorus can be suspended in polar aprotic solvent, such as dry dimethylsulfoxide (DMSO), and then mixed with deoxygenated water in order to get a molar ratio P/H₂O ranging from 14:1.5 to 3:1, more preferably of about 2:1.

Preferably, the mixture is poured in a vial and deoxygenated under vacuum and nitrogen. The vial is sealed and sonicated (37 kHz) for at least 2 days, preferably from 3 to 6 days, at temperature ranging from 20 to 40°C, preferably ranging from 25° to 35°C, more preferably about 30°C.

Afterwards, the vial is opened, added with degassed polar solvent, such as ethanol, and the suspension is centrifuged for at least 10 minutes, preferably from 20 to 60 minutes, more preferably about 30 minutes at 5,000-15,0000 rpm, preferably 7,000-12,000 rpm, more preferably about 9500 rpm.

The surnatant is removed, fresh polar aprotic solvent such as acetone is added, and the suspension is again centrifuged for at least 10 minutes, preferably from 20 to 60 minutes, more preferably about 30 minutes at 5,000-15,0000 rpm, preferably 7,000-12,000 rpm, more preferably about 9500 rpm.

The resulting dark grey powder is re-suspended by sonication (for 0.5-5 minutes, preferably 0.5-2 minutes, more preferably approximately one minute) in a polar protic solvent, such as isopropanol or methanol and the desired amount of suspension is drop-casted on a well plate and left slowly to evaporate the solvent under a stream of inert gas, such as nitrogen, in the dark to get a dry film of exfoliated black phosphorus on the surface of the well plate.

In a second aspect, the present invention relates to a pharmaceutical composition for parenteral administration comprising 2D exfoliated black phosphorus and at least one pharmaceutically acceptable excipient. Advantageously, the pharmaceutical composition of the present invention comprises or is in the form of an injectable bio-composite of 2D exfoliated black phosphorus (2D bP) with hydroxyapatite and/or polymeric materials.

According to a first embodiment, the injectable bio-composite can be prepared by adding a 2D bP in form of a suspension or solution to a polymer matrix.

The polymer matrix can be prepared by dissolving the polymer in an appropriate solvent (i.e. water, organic solvent or mixture thereof) under stirring (preferably at least 250 rpm). An aqueous solution or a suspension in an organic solvent of 2D bP is added to the polymer solution, and then the system can be maintained under stirring (preferably at least 250 rpm) until a homogeneous viscous mixture is obtained. A dialysis treatment can be performed in order to remove residual of solvent and to equilibrate the system at physiological pH value.

According to this embodiment, the process for the preparation of a bio-composite of 2D exfoliated black phosphorus in a polymeric matrix comprises the steps of (i) preparing a solution of a polymer, (ii) preparing a solution or a suspension of 2D exfoliated black phosphorus; (iii) mixing solution (i) with solution or suspension (ii); and (iv) dialyzing the mixture obtained in (iii).

The use of water in the process is preferred over the use of organic solvents.

Preferably, in the above process, a water-soluble polymer is used and an aqueous solution of the polymer and an aqueous solution of 2D exfoliated black phosphorus are prepared and mixed, thereby obtaining a viscous solution. According to a second embodiment, the injectable bio-composite can be prepared by an *in situ* synthesis of hydroxyapatite and 2D exfoliated black phosphorus.

The injectable bio-composite can be prepared by an *in situ* synthesis of HA-2D bP based on a simple mechanism where the phosphate anions derived from oxidation of the 2D bP sheets may play an essential role in anchoring calcium ions. A good nucleation of HA nanoparticles on 2D bP sheets is obtained when in situ preparation is performed at high pH value (pH>10) followed by an aging step of at least one day, preferably 2 days at a temperature in the range from 40° to 80°C, preferably from 50° to 70°C, more preferably at about 60°C.

The high pH value (pH > 10) and the reaction temperature play important roles in the degree of crystallinity, as well as the phase formation and morphology of HA as demonstrated by TEM investigation. Furthermore, during the synthesis process when the Ca²⁺ solution is added into the 2D bP solution, the Ca cations would be attracted and anchored on the oxygen atoms through electrostatic interactions and function as the nuclei for the crystallization and growth of the HA particles. The Ca²⁺ could react *in situ* with the ambient dropwise phosphate ions via electrovalent bonds to form HA nanoparticles. The high specific surface area of 2D bP is also beneficial for high loading levels of the HA particles and is helpful for forming an effective network inside the bio-composite.

More in particular, the *in situ* synthesis of HA-2D bP bio-composite comprises the steps of (i) preparation of an aqueous solution of a calcium salt, ii) adding to the solution of step (i) an aqueous solution of a phosphate and/or phosphite salts, (iii) adding to the Ca-P mixture of step (ii) an aqueous solution of 2D exfoliated black phosphorus and (iv) gelling the mixture of step (iii) by adjusting to alkaline pH.

Preferably, the calcium salt is selected from the group consisting of calcium nitrate, calcium sulphate, calcium hydrogen sulphate, calcium carbonate, calcium chloride, calcium nitrate, and combinations thereof. In preferred embodiments, the calcium salt is calcium nitrate.

Preferably, the phosphate salt is selected from the group consisting of dihydrogen phosphate, hydrogen phosphate, and phosphate salts with alkaline and/or alkaline-earth metal cations and/or ammonium or phosphonium.

Preferably, the phosphite salt is selected from the group consisting of dihydrogen phosphite, hydrogen phosphite, and phosphite salts with alkaline and/or alkaline-earth metal cations and/or ammonium or phosphonium.

Preferably, the mixture of step (ii) has a calcium to phosphorus molar ratio ranging from 1.0:1.0 to 3.0:1.0, more preferably from 1.2:1.0 to 2.0:1.0.

The pH is adjusted to a value equal to or higher than 10, preferably ranging from 10 to 12, and more preferably of about 11.

Preferably, the pH is adjusted by adding ammonium hydroxide.

Particularly, after the adjustment to alkaline pH, the aqueous solution is stirred at room temperature for at least 1 hour, more preferably for about 2 hours.

Advantageously, the process further comprises a step of aging the resulting bio-composite for at least one day, preferably two days at a temperature from 40° to 80°C, preferably from 50° to 70°C, more preferably of about 60°C. Preferably, exfoliated 2D black phosphorus obtainable by the above described process has an X-ray diffraction pattern comprising the following peaks (2θ values): 020, 040, and 060, which correspond to the reflections of black phosphorus.

According to a third embodiment, the injectable bio-composite can be prepared as an organic-inorganic composite material comprising HA-2D bP in a polymer matrix.

The bio-composites based on organic-inorganic composite materials with 2D bP can simply be obtained by adding the HA-2D bP bio-composite resulting from the *in-situ* synthesis described above to a polymer matrix.

Alternatively, bio-composites based on organic-inorganic composite materials with 2D bP can be realized by *in situ* sol-gel synthesis of hydroxyapatite in the polymer matrix.

More in particular, the *in situ* sol-gel synthesis of hydroxyapatite in the polymer matrix comprises the steps of (i) preparation of an aqueous solution of a polymer, (ii) preparation of an aqueous solution of a calcium salt, (iii) mixing the solutions of steps (i) and (ii), (iv) adding to the mixture of step (iii) an aqueous solution of a phosphate and/or phosphite salts, (v) adding to the Ca-P mixture of step (iv) an aqueous solution of 2D exfoliated black phosphorus and (vi) gelling the mixture of step (v) by adjusting to alkaline pH.

Hydroxyapatite (HA) is a calcium phosphate which is commonly denoted as Ca₅(PO₄)₃(OH), therefore belonging to the apatite group and containing a hydroxyl group (OH). For the purposes of the present invention, the term "hydroxyapatite" also encompasses, in addition to the aforesaid, all polymorphic forms, relative hydrates and solvates, in all ratios between phosphate ions, hydroxyl ions and calcium, as well as all hydroxyapatite precursors, such as amorphous calcium phosphate (ACP), octacalcium phosphate (OCP), and calcium phosphate in all polymorphic forms, relative hydrates and solvates, in all ratios between phosphate ions, hydroxyl ions and calcium.

Polymeric materials are preferably biocompatible polymers which include water-soluble polysaccharides (carbohydrates) and polypeptides, water-soluble synthetic polymers, such as poly(ethylene glycol) and poly(vinylpyrrolidone), and chain type polyesters, which are insoluble in water, but are biodegradable. The water-soluble polymers include cationic polymers, which contain amine groups or similar and are dissolved in acidic solutions to carry cations, anionic polymers, which contain carboxyl groups or the like and are dissolved in basic solutions to carry anions, and nonionic polymers, which contain hydroxyl group or the like and are well soluble in water. Illustrative examples of polymeric materials for use in the injectable composition of the present invention include gelatin, hyaluronic acid, cellulose, chitosan, collagen, fibronectin, laminin and vitronectin. Other polymeric materials that can be employed in the injectable composition of the present invention include and are not limited to poly-gamma-glutamic acid, alginate, carboxymethylcellulose, poly-L-lysine, poly-L-histidine, poly-L-arginine, polydimethylaminoethyl methacrylate, glycogen, amylose, dextran, polyacrylic acid, polymethacrylic acid, pullulan, beta-glucan, starch, hydroxyethylcellulose, carboxymethylcellulose, polyvinylpyrrolidone, polyvinyl alcohol, polyvinylpyrrolidone copolymers, polyethylene glycol, polyvinyl alkyl ether, polylactic acid, polyglycolic acid, polycaprolactone and the like. Preferred polymeric materials for use in the injectable composition of the present invention are hyaluronic acid, collagen, fibronectin, laminin and vitronectin.

The resulting bio-composite is fully injectable, without phase separation phenomena and agglomeration of inorganic phase, so that it can be advantageously used in minimally invasive surgery.

Advantageously, the pharmaceutical composition of the present invention can comprise one or more additional pharmaceutically acceptable excipient, and can be formulated according to techniques known in the art using suitable dispersing or wetting agents and suspending agents.

The pharmaceutical composition can be a sterile injectable solution or suspension of the above described bio-composites in a nontoxic parenterally acceptable diluent or solvent. Among the acceptable vehicles and solvents that can be employed are water, Ringer's solution and isotonic sodium chloride solution.

In addition, sterile, fixed oils are conventionally employed as a solvent or suspending medium (e.g., synthetic mono- or di-glycerides). Fatty acids, such as oleic acid and its glyceride derivatives are useful in the preparation of injectables, as are natural pharmaceutically-acceptable oils, such as olive oil or castor oil, especially in their polyoxyethylated versions. Other natural oils that can be employed as solvent include corn oil (maize), cottonseed oil, peanut oil, polyoxyl 35 castor oil, safflower oil, sesame oil, soybean oil, peppermint oil, coconut oil, palm seed oil, sunflower oil and hydrogenated forms thereof, or a combination thereof. Other solvents usually employed in the preparation of oil solutions or suspensions are dibutyl phthalate, diethyl phthalate, dimethyl ether, dimethyl phthalate, dimethyl sulfoxide, dimethylacetamide, ethyl acetate, ethyl alcohol (ethanol), ethyl lactate, ethyl oleate, glycerin, glycofurol, isopropyl alcohol, isopropyl myristate, isopropyl palmitate, light mineral oil, medium-chain triglycerides, methyl lactate, mineral oil, monoethanolamine, monopropylene glycol, octyldodecanol, polyethylene glycol, propylene carbonate, propylene glycol, pyrrolidone, triacetin, tricaprylin, triethanolamine, triethyl citrate, and triolein.

Illustrative examples of surfactants for use in the injectable composition of the present invention include and are not limited to CREMOPHOR^{®} EL, polyethylene glycol modified CREMOPHOR^{®} (polyoxyethyleneglyceroltriricinoleat 35), hydrogenated CREMOPHOR^{®} RH40, hydrogenated CREMOPHOR^{®} RH60, PEG-succinate, polysorbate 20, polysorbate 80, SOLUTOL^{®} HS (polyethylene glycol 660 12-hydroxystearate), sorbitan monooleate, poloxamer, LABRAFIL^{®} (ethoxylated persic oil), LABRASOL^{®} (capryl-caproyl macrogol-8-glyceride), GELUCIRE^{®} (glycerol ester), SOFTIGEN^{®} (PEG 6 caprylic glyceride), glycerin, glycol-polysorbate, or a combination thereof.

The invention will be further described in the following examples, which do not limit the scope of the invention described in the claims.

### EXAMPLE 1

### 2D black phosphorus preparation

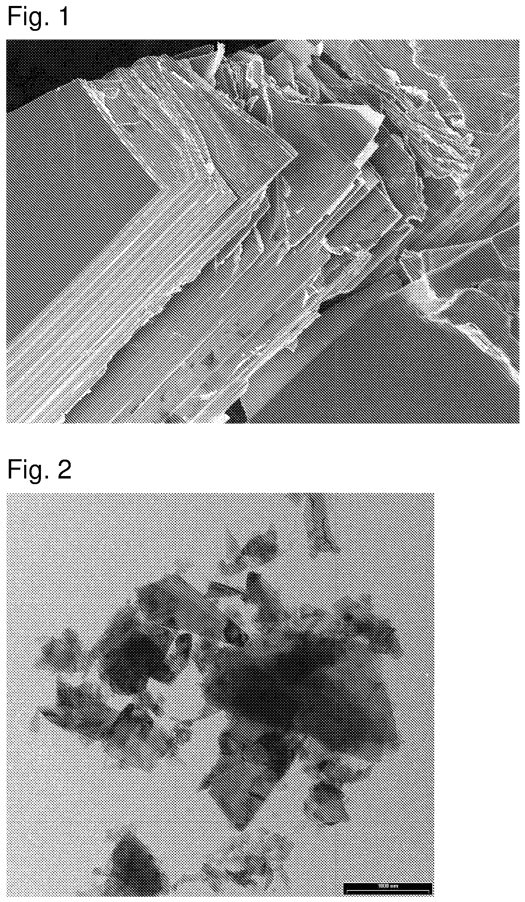

Bulk crystalline black phosphorus was prepared from red phosphorus according to the procedure disclosed by T. Nilges et al., J. Solid State Chem. 2008, 181, 1707-1711 and was characterized by scanning electron microscopy (Fig. 1), energy dispersive X-ray spectroscopy (EDX), powder X-ray diffraction and Raman spectroscopy.

Red phosphorus (99.999% purity) was purchased from Aldrich and used as received.

The SEM images were obtained from a FEI Quanta 200 FEG field-emission scanning electron microscopy with an accelerating voltage of 30 kV and a working distance of 9.1 mm.

The bulk material was subjected to liquid exfoliation under the action of ultrasounds following the procedure disclosed by M. Serrano-Ruiz et al., Adv. Mater. Interfaces 2016, 3, 1500441.

Crystals of black phosphorus (5.0 mg, 0.161 mmol) were suspended in 5.0 mL of dry DMSO and then deoxygenated water (1.0 µL, 0.056 mmol) was added in order to get a molar ratio P/H₂O = 2. The mixture was deoxygenated under vacuum and nitrogen, the vial was sealed and sonicated (37 kHz) for 4 days at 30°C. Afterwards, the vial was opened, added with degassed ethanol (15.0 mL), and the suspension was centrifuged for 30 minutes at 9500 rpm. The surnatant was removed, degassed acetone (10.0 mL) was added, and again the suspension was centrifuged at 9500 rpm for 30 min. The resulting dark grey powder was re-suspended by sonication (approximately one minute) in isopropanol (10.0 mL) and the desired amount of suspension was drop-casted on the well plate and left slowly to evaporate the solvent under a stream of nitrogen in the dark.

Exfoliated 2D black phosphorus was characterized using transmission electron microscopy (Fig. 2), energy dispersive X-ray spectroscopy (EDX), atomic force microscopy (AFM), powder X-ray diffraction (PXRD), Raman spectroscopy and X-ray photoelectron spectroscopy (XPS). Powder X-ray diffraction of the dark gray solid which settled down after centrifugation was also carried out to confirm the nature of the sample and its purity. The XRD results demonstrated the presence of only few peaks, which correspond to the 020, 040, and 060 reflections of BP, while any other peaks were absent or extremely weak.

The TEM images were obtained by using a Philips instrument operating at an accelerating voltage of 100 kV.

Raman spectra were collected with a custom-built microspectrometer. The 488 nm line from an Ar laser was focused on the sample with a 63× objective with a numerical aperture of 0.9. The power on the sample was 0.3 mW. Light emitted by the sample was dispersed by an HR550 Jobin-Yvon monochromator and the spectrum collected by a Synapse CCD.

EDX (SEM and TEM) : Bruker Quantax 800 EDX having an energy resolution of ≈130 eV, and operated with an electron beam energy of 5 kV.

X-ray powder diffraction (XRD) data were collected with an X'Pert PRO diffractometer with Cu Ka radiation ( λ = 1.5418 Å). XRD spectra were acquired at room temperature with a PANalytical X'PERT PRO diffractometer, employing Cu Ka radiation ( λ = 1.54187 Å) and a parabolic MPD-mirror for Cu radiation.

### EXAMPLE 2

### BIOLOGICAL INVESTIGATION

Biological investigations were performed using human primary osteogenic sarcoma cells (SAOS-2) and human adult osteoblasts (HOb) purchased from Sigma-Aldrich (Milan, Italy). Cells were cultured in 75 cm² cell culture flask in Dulbecco's Modified Eagle's Medium (DMEM) supplemented with 10% Fetal Bovine Serum (FBS), antibiotic solution (streptomycin 100 µg/ml and penicillin 100 U/ml, Sigma Chem. Co) and 2 mM L-glutamine, without osteogenic factors. Cells at passage 4 were used for all the experimental procedures and incubated at 37°C in a humidified atmosphere with 5% CO₂ and 95% air.

The biological investigations were performed using HOb and SAOS-2 at a density of 5x10³ in order to evaluate the effect of 2D black phosphorus on cell proliferation and osteogenic differentiation.

The SEM images were obtained from a FEI Quanta 200 FEG field-emission scanning electron microscopy with an accelerating voltage of 30 kV and a working distance of 9.1 mm.

Absorbance measurements were carried out by UV-Vis spectrophotometer Victor X3 Multilabel Plate Reader, Perkin Elmer.

Immuno-fluorescence analysis was carried out by Leica TCS sp8 confocal microscope.

Fluorescence measurements were performed using a microplate reader, Victor X3, Perkin Elmer, with the excitation wavelength of 485 nm and the emission wavelength of 538 nm.

Optical images were acquired using Motic AE31 Inverted Biological Microscope.

Alamar blue assay was purchased from AbD Serotec, Milan, Italy.

SensoLyte^{™} pNPP Alkaline Phosphatase Assay Kit was obtained from AnaSpec, DBA, Italy.

PicoGreen_dsDNA quantification kit was purchased from Invitrogen, USA.

### Example 2.1 - Cytotoxic effect

The cytotoxic effect of bulk red and black phosphorous was verified and compared.

The study was conducted at different concentration, in the range from 10 to 500 µg/ml, by using murine fibroblast cell line (L929) and human mesenchymal stem (5000 cells/well).

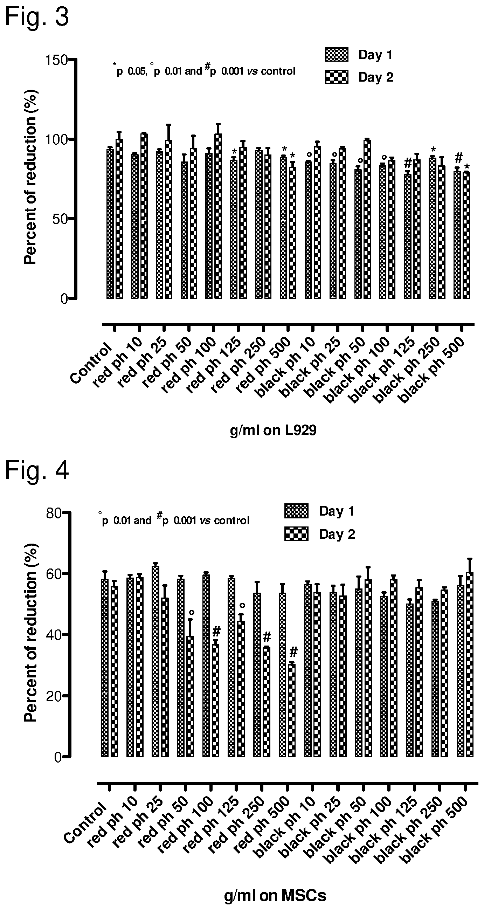

The results are illustrated in Figs. 3-4. Fig. 3 shows the effect of red and black phosphorus in DMSO at different concentration on L929 (murine fibroblasts) viability after 1 and 2 days of exposure. Fig. 4 shows the effect of red and black phosphorus in DMSO at different concentration on MSC viability after 1 and 2 days of exposure.

The results demonstrated that bulk red and black phosphorus do not show any negative effect on murine fibroblast cells proliferation.

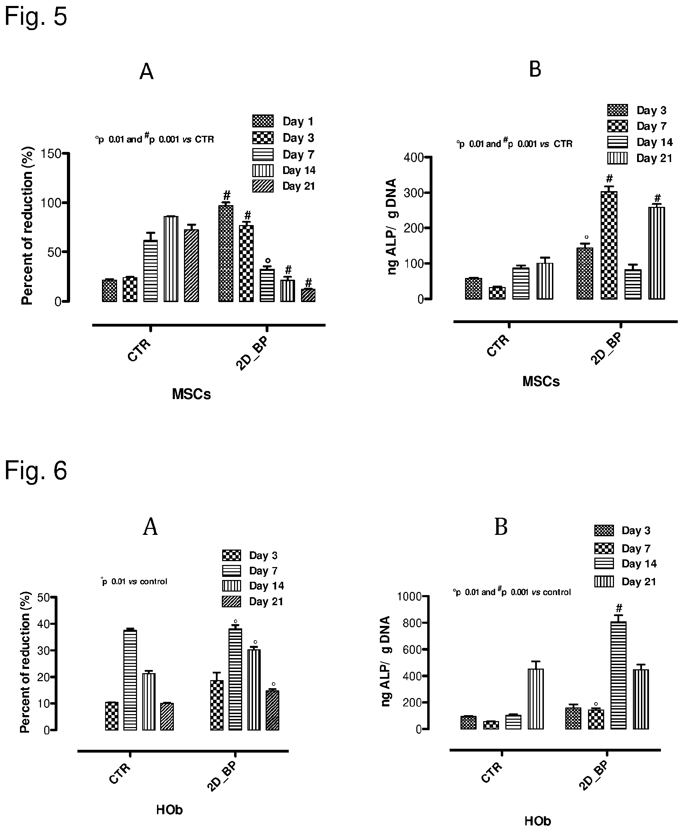

Meanwhile a different behavior was observed for hMSC; in fact, a reduction in cell proliferation was obtained for bulk red phosphorous starting from concentration 50 µg/mL after 48 hours of incubation. By contrast, bulk black phosphorus increased hMSC cell proliferation by increasing its concentration from 50 to 500 µg/mL after 48 hours of exposure. Furthermore, the cytotoxicity of 2D exfoliated black phosphorus (2D bP) was also investigated on human mesenchymal stem cells. The results are illustrated in Fig. 5. Fig. 5 shows the effect of 2D exfoliated black phosphorus on MSC viability after 3, 7 and 14 days of exposure (A) and the alkaline phosphatase activity (B) results after 3, 7, 14 and 21 days of cell cultures.

No cytotoxic effects were observed on MSCs in presence of 2D exfoliated black phosphorus using Alamar Blue assay. ALP expression increases in presence of 2D exfoliated black phosphorus at days 3, 7 and 21.

### Example 2.2 - Cell proliferation

Cell proliferation was assessed by Alamar blue assay, a commonly used tool that allows to control the cell proliferation by measuring the metabolic activity of live cells, as disclosed by J. O'Brien et al., Eur. J. Biochem. 2000, 267, 5421-5426.

For Alamar blue assay HOb and SAOS-2 were seeded on 96-well plate coated with 2D black phosphorus. As control, the same cell lines were deposited on a well plate without coating. The cell culture medium was removed after 3, 7, 14 and 21 days of culture time and Alamar Blue^{™} reagent was added directly to each well. All plates were incubated at 37°C for 4 hours to allow cells to convert resazurin to resorufin and the optical density was immediately measured with a UV-Vis spectrophotometer at wavelengths of 570 and 600 nm. Empty wells were used to correct any background interference due to the redox indicator.

The results showed that 2D black phosphorus significantly increases HOb cell proliferation over culture time compared to control consisting in cells seeded on a polystyrene plate surface (Fig. 6A).

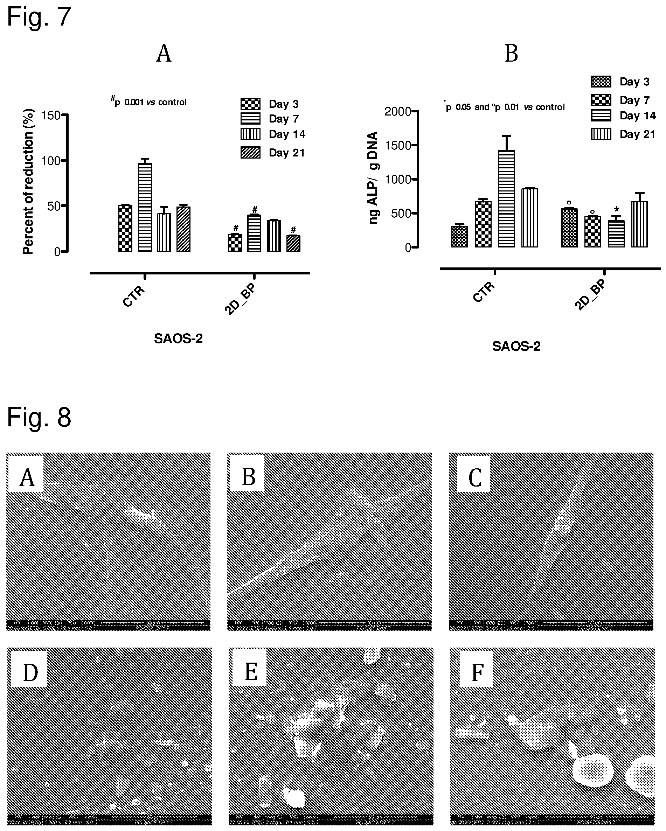

Different behavior of 2D black phosphorus as substrate was observed for osteosarcoma cells (SAOS-2), since in the presence of 2D black phosphorus, the cell proliferation significantly decreased over culture time compared to control (Fig. 7A).

These results confirmed that 2D black phosphorus not only contributes to inhibit cancer progression but also induces new bone tissue formation due to its oxidation to phosphate anions that, combined with calcium cations, represent hydroxyapatite (the major component of bone) precursors. Thus, after cancer resection, 2D black phosphorus exerts anti-proliferative effects on cancer cells and simultaneously promotes induction of newly forming bone tissue development by stimulating pre-osteoblast differentiation.

### Example 2.3 - ALP expression

The metalloenzyme known as alkaline phosphatase (ALP) has several different functions in the tissues where it occurs; for instance, in bone tissue ALP has a crucial role in the mineralization process, thus representing a marker of osteogenic differentiation.

Here, ALP expression was assessed on HOb and SAOS-2 cell lysates (50 µl) by measuring the activity of ALP enzyme which catalyzes the cleavage of a phosphate group and releases p-nitrophenol from p-nitrophenyl phosphate using the SensoLyteTM pNPP Alkaline Phosphatase Assay Kit.

The absorbance was measured at a wavelength of 405 nm on a plate reader to determine enzyme concentrations per construct at 3, 7, 14 and 21 days of cell culture. The ALP values were corrected on DNA micrograms of cells present in each well. DNA values were measured by using PicoGreen_dsDNA quantification kit.

The ALP results were reported as nanograms of ALP normalized to the micrograms of total DNA content (ng ALP/µg DNA).

The results showed that 2D black phosphorus induced a higher ALP expression than control, in particular a plateau effect on ALP expression at day 14 of cell culture was observed (Fig. 6B).

Moreover, we have found that the presence of 2D black phosphorus was able to inhibit the ALP expression in SAOS-2 cells (Fig. 7B), thus indicating the ability of 2D black phosphorus as substrate to block the metabolic activity of osteosarcoma derived cancer cells.

### Example 2.4 - Cell attachment and spreading

Cell attachment and spreading on the 2D black phosphorus surface were evaluated by SEM analysis. For this purpose, HOb and SAOS-2 cells were seeded on 2D black phosphorus and incubated for 7 days at 37 °C. After 7 days of incubation, the cells were fixed with a solution of 4% paraformaldehyde in PBS for 24 hours at 4°C. Later, samples were washed, dehydrated using a series of increasing ethanol concentration and fixed by means of a double adhesive tape to aluminum stubs applying a tension of 20 kV. The stubs were sputter-coated with gold to reach a thickness of around 20 nm (Sputter coater Emitech K575).

The cytotoxic effects on SAOS-2 were confirmed by SEM images, where the initial characteristic morphology of the cells disappeared and was replaced by a typical shape of apoptotic cells (Figs. 8D-F). By contrast, SEM images of HOb (Figs. 8A-C) showed a good cell spreading and the polygonal morphology of differentiating cells.

### Example 2.5 - Cell morphology

Cell morphology was also studied by immunofluorescence analysis using a confocal laser scanning microscopy.

For this purpose, HOb and SAOS-2 cells were cultured in chamber slides coated with 2D black phosphorus for 7 days at a density of 2x10⁴ cells/slide.

After this time, the cells were fixed in 4% paraformaldehyde in Phosphate Buffer Solution (PBS 1X) at room temperature for 10-30 minutes, washed thrice with PBS and incubated with 0.1% Triton X-100 in PBS for 1 hour to increase permeability. Then cells were washed 2-3 times in PBS and phalloidin-conjugate working solution was added to each slides and incubated at room temperature for 30-90 minutes.

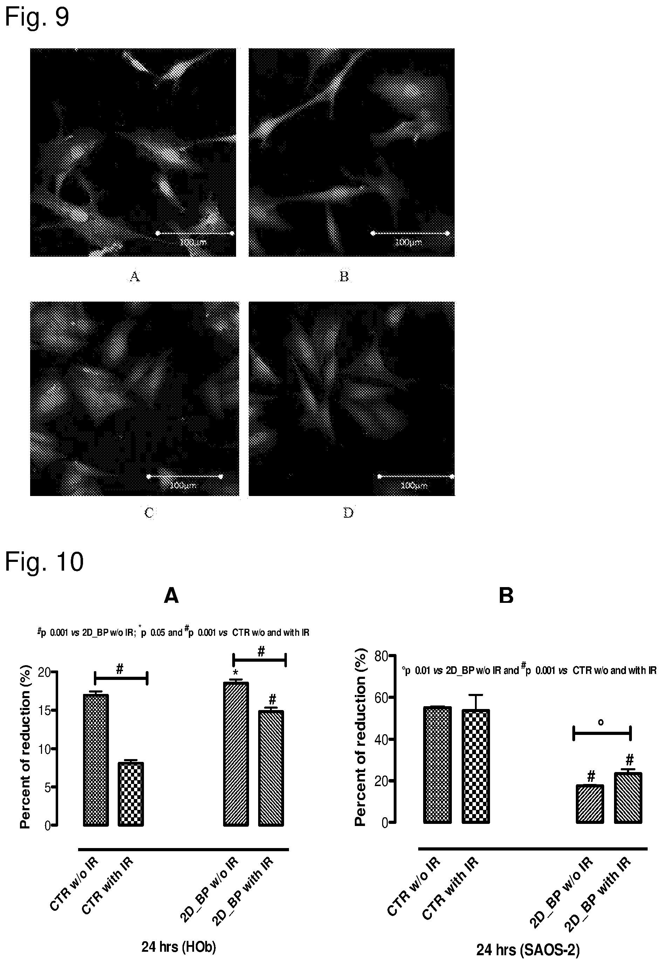

Finally, slides were rinsed 2-3 times with PBS and cells were observed by confocal laser scanning microscopy. HOb showed a good morphology (Figs. 9A,9B). Conversely, SAOS-2 appeared to lose their characteristic morphology as suffering cells in the presence of 2D black phosphorus (Figs. 9C,9D).

### Example 2.6 - ROS production

The effect of 2D black phosphorus on Reactive Oxygen Species (ROS) was evaluated on HOb and SAOS-2 cells with and without stimulation by near-infrared light irradiation.

In the former case, biological response of cells seeded onto 2D black phosphorus, in terms of cell proliferation and ROS production was measured after two irradiation cycles of 15 minutes each.

After 24 hours of interaction between exfoliated black phosphorus and the cells, the latter were firstly irradiated for 15 minutes and then incubated overnight at 37°C. After this time, a second stimulation was performed for the same time and cell proliferation was tested by Alamar Blue assay. At the same time, ROS species production was detected by using the fluorescent probe DCFH-DA. The latter in presence of ROS, is rapidly oxidized to highly fluorescent dichlorofluorescein (DCF) and its fluorescence intensity is proportional to the amount of ROS formed intracellularly. Concerning the analysis of ROS production, cells were incubated for 1 hour with 100 µM solution of DCFH-DA in Hanks' balanced salt solution containing 1% FBS; then cells were washed thrice with PBS and incubated with the Fenton's reagent (H₂O₂/Fe²⁺ 2 mM) for 3 hours at 37°C. The DCF fluorescence intensity was estimated by using a fluorescent microplate reader with the excitation wavelength of 485 nm and the emission wavelength of 538 nm. The same procedure was adopted for the experiment without near-infrared light irradiation, but for the irradiation cycles of 15 minutes each.

The results suggested that IR had no effect on HOb proliferation because 2D black phosphorus without infrared stimulation promoted the HOb viability, thus inducing higher proliferation values than control (Fig. 10A). Conversely, 2D black phosphorus with and without IR, induced a significant reduction in SAOS-2 proliferation (Fig. 10B).

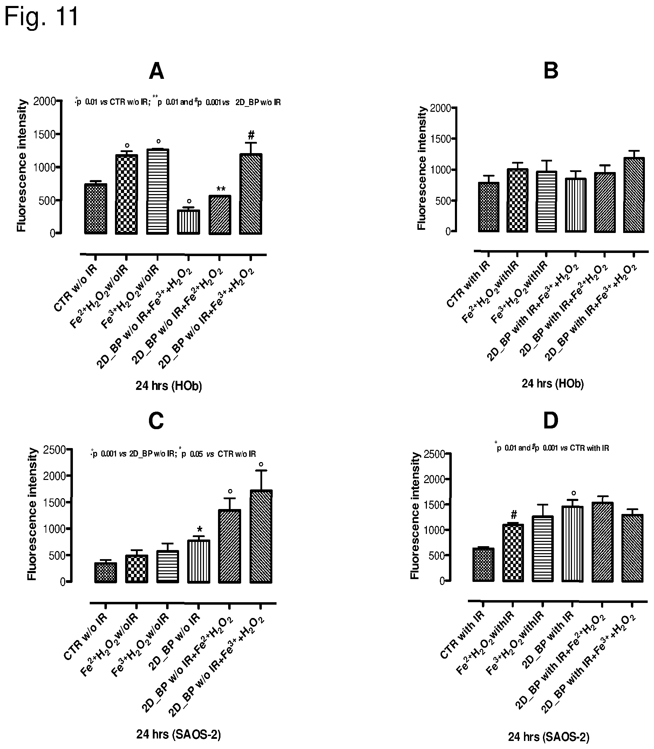

The measurement of ROS production after 24 hours due to the stimulation caused by the Fenton reagent, indicated that on healthy HOb the presence of 2D black phosphorus without irradiation reduced the ROS production, suggesting potential antioxidant properties (Fig. 11A). In contrast, near-infrared stimulation had not significant effects on ROS generation (Fig. 11B). In the case of osteosarcoma derived cells (SAOS-2), the presence of 2D black phosphorus doubled the ROS production compared to the reference experiment and the ROS generation increased further in combination with IR (Figs. 11C-D).

The overall results on cell proliferation pointed out that 2D black phosphorus can inhibit cell proliferation on in vitro model of osteosarcoma, even without photo-thermal treatment. Moreover, the data obtained on HOb cells suggested that this substrate may also exert a protective effect on healthy precursors of bone mature cells.

### Example 2.7 - Effect of 2D exfoliated black phosphorus on cancer-related inflammation

In order to test the effect of 2D exfoliated black phosphorus on cancer-related inflammation, a co-culture model was realized by seeding SAOS-2 and HOb at density of 5x10⁴ respectively in a 6-multiwell plate in presence and in absence of 2D exfoliated black phosphorus.

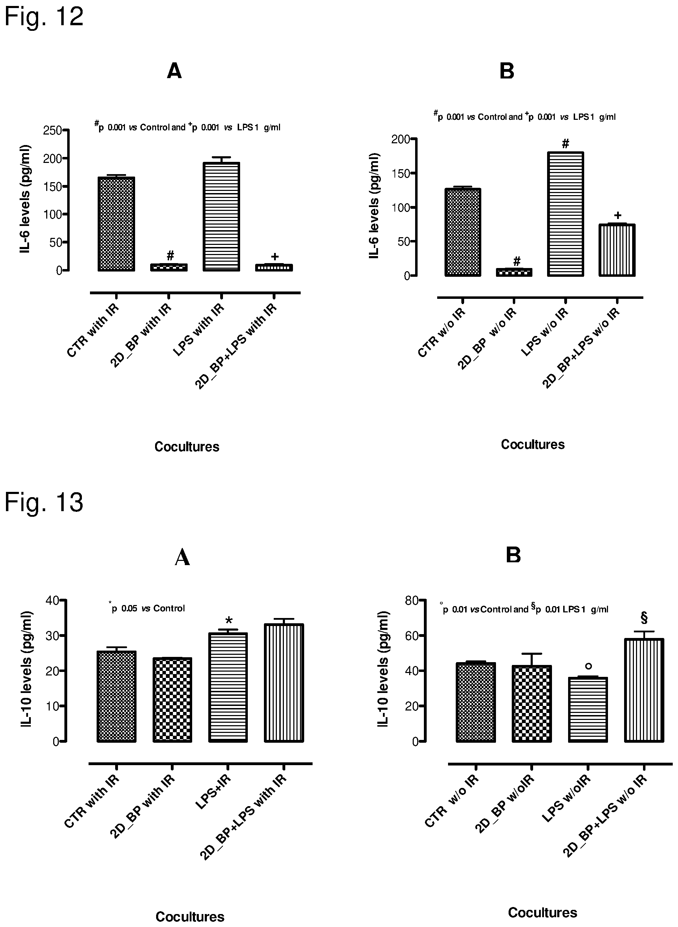

After 24 hours, the cells were firstly irradiated for 15 minutes and then incubated overnight at 37°C. After this time, inflammation was induced by LPS at concentration of 1µg/ml and later a second irradiation was performed for other 15 minutes. As an *in vitro* model of acute inflammation, 3 days after LPS stimulation interleukin-6 (IL-6) and interleukin-10 (IL-10) levels in cell supernatants were quantified using commercial ELISA kit. IL-6 is a proinflammatory cytokine that promotes in vitro proliferation of SAOS-2
The results showed that LPS stimulation caused a significant increase in IL-6 values (Figs. 12A-B) but highest IL-6 values were observed for LPS without IR stimulation (Fig. 12B). The interaction between cells and 2D exfoliated black phosphorus significantly decreased IL-6 levels induced by LPS after 3 days of stimulation as model of acute inflammation (Fig. 12A, 12B). The reduction of IL-6 levels confirmed the previously described cytotoxic effect of 2D exfoliated black phosphorus on SAOS-2 cells. The lower values of IL-6 in cells treated with LPS obtained with IR stimulation is due to the anti-inflammatory properties of Near Infrared (NIR) based therapy.

The results not only demonstrated an inhibitory effect of 2D exfoliated black phosphorus on pro-inflammatory mediators such as IL-6 but also suggested an anti-inflammatory potential of 2D exfoliated black phosphorus because of its ability to increase IL-10 levels (Figs. 13A-B), a well-known anti-inflammatory cytokine. As shown in Figs. 13A-B, IR treatment blocks LPS action thus inducing the same values of IL-10 in control cells and stimulated cells with LPS. By contrast in plate without IR stimulation, LPS induced a reduction in IL-10 levels compared to control and 2D exfoliated black phosphorus significantly increased these IL-10 levels thus suggesting an anti-inflammatory activity.

### Example 2.8 - Effect of 2D exfoliated black phosphorus on nitrites

Nitrites, stable metabolites of nitric oxide, are used as index of oxidative stress during inflammatory processes. Here the effect of 2D exfoliated black phosphorus on basal nitrite levels induced by LPS at concentration of 1µg/ml was analyzed using Griess assay.

For this purpose, SAOS-2 and HOb were seeded in a 6-multiwell plate at density of 5x10⁴ cells respectively. After 3 days of incubation, 100 µL of supernatant was transferred into a 96-well plate and an equal volume of Griess reagent was added to each well. The samples were incubated for 1 hour at room temperature and the absorbance was measured at a wavelength of 550 nm.

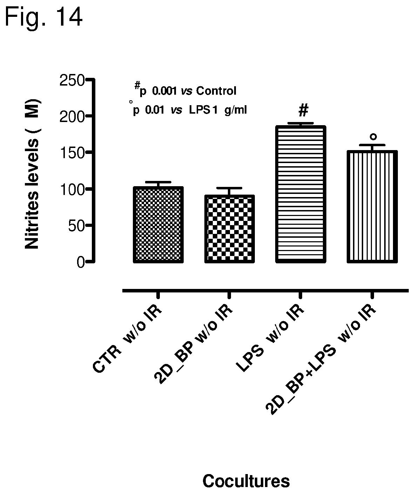

The results showed that LPS produced a significant increase in nitrite production compared to control (Fig. 14). Conversely treatment with 2D exfoliated black phosphorus caused a significant reduction in LPS induced nitrite production (Fig. 14).

### EXAMPLE 3

### Preparation of the bio-composites

### Preparation of 2D bP-polymer bio-composite

The injectable bio-composite can be developed by adding a 2D bP solution in a polymer matrix. The polymer solution (i) was prepared by dissolving the polymer in appropriate solvent (i.e. distilled water or organic solvent) by stirring at 200 rpm. 2D bP solution was added to (i) and then the system was kept under stirring (200 rpm) until a homogeneous viscous solution was obtained. The dialysis treatment can be performed in order to remove residual of solvent and to equilibrate the system at physiological pH value. A 2D bP solution was obtained by ultrasonication of an appropriate amount of black phosphorus (depending of composition) in distilled water or in a mixture of DMSO and H₂O for 30 minutes.

### Preparation of HA-2D bP bio-composite

Moreover, bio-composite based on hydroxyapatite-2D bP was also synthesized by sol-gel approach. More in particular, the *in situ* synthesis of HA-2D bP bio-composite comprises the steps of (i) preparation of an aqueous solution of a calcium salt, ii) adding to the solution of step (i) an aqueous solution of a phosphate and/or phosphite salts, (iii) adding to the Ca-P mixture of step (ii) an aqueous solution of 2D exfoliated black phosphorus and (iv) gelling the mixture of step (iii) by adjusting to alkaline pH.

### Preparation of HA-2D bP-polymer bio-composite

Furthermore, bio-composites based on organic-inorganic composite materials with 2D bP was fabricated by *in situ* sol-gel synthesis of hydroxyapatite in the polymer matrix (i.e. gelatin, hyaluronic acid, cellulose, and the like). Briefly, viscous polymer solution was prepared in distilled water, dH₂O at 40°C with constant adjustments of the pH (10-10.5) by using NH₄OH solution (28.0-30.0% NH₃ basis). Simultaneously, precursor of calcium (i.e. calcium nitrate Ca(NO₃)₂x4H₂O) and phosphorous (i.e. ammonium hydrogen phosphate (NH₄)₂HPO₄ or phosphorous pentoxide P₂O₅) were dissolved in distilled water (the first) or ethanol (the second), and stirred for 2 hours.

The Ca²⁺ solution was added to polymer solution and mixed at 250 rpm for 2 hours at 40°C; then a phosphate solution was introduced to the Ca²⁺/Polymer system. The alkaline environment was reached by using NH₄OH solution. The 2D bP solution (at different concentration) was added to the system in order to obtain injectable materials at different compositions. The samples were treated at 40°C for 24 hours (stirring at 85 rpm) and sonicated for 3 hours to obtain a homogeneous system.

The present invention has been disclosed with particular reference to some specific embodiments thereof, but it should be understood that modifications and changes may be made by the persons skilled in the art without departing from the scope of the invention as defined in the appended claims.

## Claims

1. 2D exfoliated black phosphorus for use in the treatment of bone cancer without phototherapy.

2. 2D exfoliated black phosphorus for use according to claim 1, wherein said bone cancer is osteosarcoma, Ewing's family of tumors, chondrosarcoma, malignant giant cell tumor, malignant fibrous histiocytoma, and adamantinoma.

3. 2D exfoliated black phosphorus for use according to claim 1, wherein said bone cancer is secondary bone cancer metastasized from other tissues, including breast, lung, prostate, and kidney.

4. Pharmaceutical composition for use in the treatment of bone cancer without phototherapy by parenteral administration comprising 2D exfoliated black phosphorus and at least one pharmaceutically acceptable excipient.

5. Pharmaceutical composition for use according to claim 4, wherein said composition comprises an injectable bio-composite of 2D exfoliated black phosphorus (2D bP) with hydroxyapatite or a polymeric material.

6. Pharmaceutical composition for use according to claim 5, wherein said composition comprises an injectable bio-composite of 2D exfoliated black phosphorus (2D bP) with hydroxyapatite and a polymeric material.

7. Pharmaceutical composition for use according to claim 6, wherein said polymeric material is selected from the group consisting of water-soluble polysaccharides (carbohydrates), water-soluble polypeptides, water-soluble synthetic polymers, and biodegradable chain type polyesters.

8. Pharmaceutical composition for use according to claim 7, wherein said polymeric material is selected from the group consisting of gelatin, hyaluronic acid, cellulose, chitosan, collagen, fibronectin, laminin and vitronectin.

9. Process for the preparation of a bio-composite of 2D exfoliated black phosphorus in a polymeric matrix comprising the following steps of:
(i) preparing a solution of a polymer selected from the group consisting of gelatin, hyaluronic acid, chitosan, collagen, fibronectin, laminin and vitronectin,
(ii) preparing a aqueous solution or suspension of 2D exfoliated black phosphorus, and
(iii) mixing solution (i) with solution (ii); and
(iv) dialyzing the mixture obtained in (iii).

10. Process for the preparation of a bio-composite of 2D exfoliated black phosphorus with hydroxyapatite comprising the following steps of:
(i) preparing an aqueous solution of a calcium salt,
(ii) adding to the solution of step (i) an aqueous solution of a phosphate and/or phosphite salts,
(iii) adding to the Ca-P mixture of step (ii) an aqueous solution of 2D exfoliated black phosphorus, and
(iv) gelling the mixture of step (iii) by adjusting to alkaline pH equal to or higher than 10.

11. Process for the preparation of a bio-composite according to claim 10, further comprising the step of (v) adding a solution of a polymer.

12. Process for the preparation of a bio-composite of 2D exfoliated black phosphorus with hydroxyapatite in a polymeric matrix comprising the following steps of:
(i) preparing an aqueous solution of a polymer selected from gelatin, hyaluronic acid, cellulose, chitosan, collagen, fibronectin, laminin and vitronectin,
(ii) preparing an aqueous solution of a calcium salt,
(iii) mixing the solutions of steps (i) and (ii),
(iv) adding to the mixture of step (iii) an aqueous solution of a phosphate and/or phosphite salts,
(v) adding to the Ca-P mixture of step (iv) an aqueous solution of 2D exfoliated black phosphorus, and
(vi) gelling the mixture of step (v) by adjusting to alkaline pH equal to or higher than 10.

13. Pharmaceutical composition for parenteral administration comprising an injectable bio-composite of 2D exfoliated black phosphorus (2D bP) with hydroxyapatite and a polymeric material selected from the group consisting of hyaluronic acid, collagen, fibronectin, laminin and vitronectin.

## Patentansprüche

1. 2D-exfolierter schwarzer Phosphor zur Verwendung bei der Behandlung von Knochenkrebs ohne Phototherapie.

2. 2D-exfolierter schwarzer Phosphor zur Verwendung nach Anspruch 1, wobei der Knochenkrebs ein Osteosarkom, ein Tumor aus der Ewing-Tumorfamilie, ein Chondrosarkom, ein maligner Riesenzelltumor, ein malignes fibröses Histiozytom oder ein Adamantinom ist.

3. 2D-exfolierter schwarzer Phosphor zur Verwendung nach Anspruch 1, wobei der Knochenkrebs ein sekundärer Knochenkrebs ist, der aus anderen Geweben, einschließlich Brust, Lunge, Prostata und Niere, metastasiert ist.

4. Pharmazeutische Zusammensetzung zur Verwendung bei der Behandlung von Knochenkrebs ohne Phototherapie durch parenterale Verabreichung, umfassend 2D-exfolierten schwarzen Phosphor und mindestens einen pharmazeutisch annehmbaren Hilfsstoff.

5. Pharmazeutische Zusammensetzung zur Verwendung nach Anspruch 4, wobei die Zusammensetzung einen injizierbaren Bioverbundstoff aus 2D-exfoliertem schwarzem Phosphor (2D bP) mit Hydroxylapatit oder einem polymeren Material umfasst.

6. Pharmazeutische Zusammensetzung zur Verwendung nach Anspruch 5, wobei die Zusammensetzung einen injizierbaren Bioverbundstoff aus 2D-exfoliertem schwarzem Phosphor (2D bP) mit Hydroxylapatit und einem polymeren Material umfasst.

7. Pharmazeutische Zusammensetzung zur Verwendung nach Anspruch 6, wobei das polymere Material ausgewählt ist aus der Gruppe bestehend aus wasserlöslichen Polysacchariden (Kohlenhydraten), wasserlöslichen Polypeptiden, wasserlöslichen synthetischen Polymeren und biologisch abbaubaren Kettenpolyestern.

8. Pharmazeutische Zusammensetzung zur Verwendung nach Anspruch 7, wobei das polymere Material ausgewählt ist aus der Gruppe bestehend aus Gelatine, Hyaluronsäure, Cellulose, Chitosan, Kollagen, Fibronektin, Laminin und Vitronektin.

9. Verfahren zur Herstellung eines Bioverbundstoffs aus 2D-exfoliertem schwarzem Phosphor in einer polymeren Matrix, umfassend die folgenden Schritte:
(i) Herstellen einer Lösung eines Polymers, ausgewählt aus der Gruppe bestehend aus Gelatine, Hyaluronsäure, Chitosan, Kollagen, Fibronektin, Laminin und Vitronektin,
(ii) Herstellen einer wässrigen Lösung oder Suspension von 2D-exfoliertem schwarzem Phosphor, und
(iii) Mischen der Lösung aus Schritt (i) mit der Lösung aus Schritt (ii) und
(iv) Dialysieren des in Schritt (iii) erhaltenen Gemischs.

10. Verfahren zur Herstellung eines Bioverbundstoffs aus 2D-exfoliertem schwarzem Phosphor mit Hydroxylapatit, umfassend die folgenden Schritte:
(i) Herstellen einer wässrigen Lösung eines Calciumsalzes,
(ii) Zugeben einer wässrigen Lösung von Phosphat- und/oder Phosphitsalzen zu der Lösung aus Schritt (i),
(iii) Zugeben einer wässrigen Lösung von 2D-exfoliertem schwarzem Phosphor zu dem Ca-P-Gemisch aus Schritt (ii) und
(iv) Gelieren des Gemischs aus Schritt (iii) durch Einstellen auf einen alkalischen pH-Wert von 10 oder höher.

11. Verfahren zur Herstellung eines Bioverbundstoffs nach Anspruch 10, ferner umfassend den folgenden Schritt:
(v) Zugeben einer Lösung eines Polymers.

12. Verfahren zur Herstellung eines Bioverbundstoffs aus 2D-exfoliertem schwarzem Phosphor mit Hydroxylapatit in einer polymeren Matrix, umfassend die folgenden Schritte:
(i) Herstellen einer wässrigen Lösung eines Polymers, ausgewählt aus Gelatine, Hyaluronsäure, Cellulose, Chitosan, Kollagen, Fibronektin, Laminin und Vitronektin,
(ii) Herstellen einer wässrigen Lösung eines Calciumsalzes,
(iii) Mischen der Lösungen aus den Schritten (i) und (ii),
(iv) Zugeben einer wässrigen Lösung von Phosphat- und/oder Phosphitsalzen zu dem Gemisch aus Schritt (iii),
(v) Zugeben einer wässrigen Lösung von 2D-exfoliertem schwarzem Phosphor zu dem Ca-P-Gemisch aus Schritt (iv) und
(vi) Gelieren des Gemischs aus Schritt (v) durch Einstellen auf einen alkalischen pH-Wert von 10 oder höher.

13. Pharmazeutische Zusammensetzung zur parenteralen Verabreichung, umfassend einen injizierbaren Bioverbundstoff aus 2D-exfoliertem schwarzem Phosphor (2D bP) mit Hydroxylapatit und einem polymeren Material, ausgewählt aus der Gruppe bestehend aus Hyaluronsäure, Kollagen, Fibronektin, Laminin und Vitronektin.

## Revendications

1. Phosphore noir 2D exfolié pour l'utilisation dans le traitement du cancer des os sans photothérapie.

2. Phosphore noir 2D exfolié pour l'utilisation selon la revendication 1, dans lequel ledit cancer des os est l'ostéosarcome, la famille des tumeurs de Ewing, le chondrosarcome, la tumeur maligne à cellules géantes, l'histiocytome fibreux malin, et l'adamantinome.

3. Phosphore noir 2D exfolié pour l'utilisation selon la revendication 1, dans lequel ledit cancer des os est un cancer secondaire des os métastasé à partir d'autres tissus, incluant le sein, le poumon, la prostate, et le rein.

4. Composition pharmaceutique pour l'utilisation dans le traitement du cancer des os sans photothérapie par administration parentérale comprenant du phosphore noir 2D exfolié et au moins un excipient pharmaceutiquement acceptable.

5. Composition pharmaceutique pour l'utilisation selon la revendication 4, dans laquelle ladite composition comprend un bio-composite injectable de phosphore noir 2D exfolié (2D bP) avec de l'hydroxyapatite ou un matériau polymère.

6. Composition pharmaceutique pour l'utilisation selon la revendication 5, dans laquelle ladite composition comprend un bio-composite injectable de phosphore noir 2D exfolié (2D bP) avec de l'hydroxyapatite et un matériau polymère.

7. Composition pharmaceutique pour l'utilisation selon la revendication 6, dans laquelle ledit matériau polymère est sélectionné dans le groupe constitué des polysaccharides solubles dans l'eau (hydrates de carbone), des polypeptides solubles dans l'eau, des polymères synthétiques solubles dans l'eau, et des polyesters de type à chaîne biodégradable.

8. Composition pharmaceutique pour l'utilisation selon la revendication 7, dans laquelle ledit matériau polymère est sélectionné dans le groupe constitué de la gélatine, de l'acide hyaluronique, de la cellulose, du chitosane, du collagène, de la fibronectine, de la laminine et de la vitronectine.

9. Procédé de préparation d'un bio-composite de phosphore noir 2D exfolié dans une matrice polymère comprenant les étapes suivantes consistant à :
(i) préparer une solution d'un polymère sélectionné dans le groupe constitué de la gélatine, de l'acide hyaluronique, du chitosane, du collagène, de la fibronectine, de la laminine et de la vitronectine,
(ii) préparer une solution ou une suspension aqueuse de phosphore noir 2D exfolié, et
(iii) mélanger la solution (i) avec la solution (ii) ; et
(iv) dialyser le mélange obtenu dans (iii).

10. Procédé de préparation d'un bio-composite de phosphore noir 2D exfolié avec de l'hydroxyapatite comprenant les étapes suivantes consistant à :
(i) préparer une solution aqueuse d'un sel de calcium,
(ii) ajouter à la solution de l'étape (i) une solution aqueuse de sels de phosphate et/ou de phosphite,
(iii) ajouter au mélange de Ca-P de l'étape (ii) une solution aqueuse de phosphore noir 2D exfolié, et
(iv) gélifier le mélange de l'étape (iii) en ajustant au pH alcalin égale ou supérieure à 10.

11. Procédé de préparation d'un bio-composite selon la revendication 10, comprenant en outre l'étape consistant à (v) ajouter une solution d'un polymère.

12. Procédé de préparation d'un bio-composite de phosphore noir 2D exfolié avec de l'hydroxyapatite dans une matrice polymère comprenant les étapes suivantes consistant à :
(i) préparer une solution aqueuse d'un polymère sélectionné parmi la gélatine, l'acide hyaluronique, la cellulose, le chitosane, le collagène, la fibronectine, la laminine et la vitronectine,
(ii) préparer une solution aqueuse d'un sel de calcium,
(iii) mélanger les solutions des étapes (i) et (ii),
(iv) ajouter au mélange de l'étape (iii) une solution aqueuse de sels de phosphate et/ou de phosphite,
(v) ajouter au mélange de Ca-P de l'étape (iv) une solution aqueuse de phosphore noir 2D exfolié, et
(vi) gélifier le mélange de l'étape (v) en ajustant au pH alcalin égale ou supérieure à 10.

13. Composition pharmaceutique pour l'administration parentérale comprenant un bio-composite injectable de phosphore noir 2D exfolié (2D bP) avec de l'hydroxyapatite et un matériau polymère sélectionné dans le groupe constitué de l'acide hyaluronique, du collagène, de la fibronectine, de la laminine et de la vitronectine.
